(19) Europäisches Patentamt
European Patent Office
Office européen des brevets

(11) Veröffentlichungsnummer : **0 180 544**
**B1**

(12) # EUROPÄISCHE PATENTSCHRIFT

(45) Veröffentlichungstag der Patentschrift : **15.06.88**

(21) Anmeldenummer : **85810480.5**

(22) Anmeldetag : **21.10.85**

(51) Int. Cl.⁴ : **C 07 D209/42**, C 07 D241/18, C 07 D487/04 // (C07D487/04, 241:00, 209:00)

(54) **Verfahren zur Herstellung einer Carbonsäure.**

(30) Priorität : **26.10.84 US 664916**

(43) Veröffentlichungstag der Anmeldung : **07.05.86 Patentblatt 86/19**

(45) Bekanntmachung des Hinweises auf die Patenterteilung : **15.06.88 Patentblatt 88/24**

(84) Benannte Vertragsstaaten : **CH DE FR GB IT LI NL**

(56) Entgegenhaltungen :
**EP-A- 0 050 850**
**EP-A- 0 096 517**
**DE-A- 2 114 230**
**DE-A- 2 162 422**
**DE-A- 2 354 056**
**DE-B- 1 965 981**

(73) Patentinhaber : **CIBA-GEIGY AG**
**Klybeckstrasse 141**
**CH-4002 Basel (CH)**

(72) Erfinder : **Roloff, Achim, Dr.**
**Waldshuterstrasse 55**
**CH-4310 Rheinfelden (CH)**
Erfinder : **Gschwend, Heinz Werner, Dr.**
**112 Sherwood Drive**
**New Providence New Jersey (US)**

**EP 0 180 544 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein neues Verfahren zur Herstellung von enantiomerenreiner 2S- oder 2R-Indolincarbonsäure der Formel

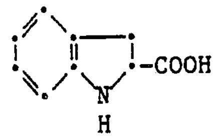

(I)

und ihrer Salze. Dieser chirale Heterocyclus ist ein wichtiger Synthesebaustein für die Herstellung einiger pharmakologisch sehr wertvoller Verbindungen, wie beispielsweise der 1-(4-Ethoxycarbonyl-2R,4R-dimethylbutanoyl)-2S-indolincarbonsäure, siehe z. B. EP-A-0 050 850. Dort wird unter Verwendung von Verbindungen der Formel I die Herstellung hochwirksamer ACE-Hemmer beschrieben. Die Bereitstellung der reinen Enantiomere der Formel I gelang bisher nur auf dem Wege einer Racemattrennung unter Inkaufnahme der mit einer solchen Trennoperation verbundenen Nachteile. Das Verfahren der vorliegenden Erfindung vermeidet die Nachteile einer Racemattrennung, wie insbesondere die mit der Trennung verbundenen Substanzverluste. Die stereochemisch einheitliche Orientierung am C2-Atom einer Verbindung der Formel I wird erfindungsgemäss dadurch herbeigeführt, dass der heterocyclische Fünfring in der Formel I aus einem offenkettigen Vorläufer durch diastereoselektive Cyclisierung gebildet wird.

Im einzelnen betrifft die Erfindung ein Verfahren zur Herstellung von enantiomerenreinen Verbindungen der Formel I und ihrer Salze, dadurch gekennzeichnet, dass man eine Verbindung der Formel II,

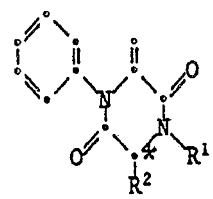

(II)

worin $R^1$ Wasserstoff, Niederalkyl oder Arylniederalkyl bedeutet, $R^2$ für unsubstituiertes oder mit Niederalkoxy, Niederalkylthio, Aryl oder Niederalkoxyaryl substituiertes Niederalkyl, oder für Aryl steht, oder worin $R^1$ und $R^2$ gemeinsam Niederalkylen bedeuten, das mit Niederalkoxy substituiert oder mit einem fünf- bis siebengliedrigen Carbocyclus kondensiert sein kann, wobei Nieder einen Gehalt von 1 bis 7 C-Atomen angibt und Aryl für 1- oder 2-Naphthyl oder Phenyl steht, und worin das Symbol * ein einheitlich in S- oder R-Konfiguration vorliegendes Kohlenstoffatom kennzeichnet, zu einer Verbindung der Formel III,

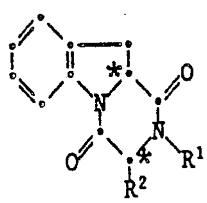

(III)

worin $R^1$ und $R^2$ die angegebenen Bedeutungen haben und die beiden Symbole * entweder beide ein in S-Konfiguration oder beide ein in R-Konfiguration vorliegendes Kohlenstoffatom kennzeichnen, diastereoselektiv photochemisch cyclisiert, diese verbindung hydrolysiert und aus dem Hydrolysat die Verbindung der Formel I, gegebenenfalls in Form eines Salzes, abtrennt und gegebenenfalls eine erhaltene freie Verbindung der Formel I in ein Salz oder ein Salz in ein anderes Salz oder in die freie Verbindung der Formel I umwandelt.

Die vor- und nachstehend verwendeten Allgemeinbegriffe haben, sofern sie nicht abweichend definiert sind, die folgenden Bedeutungen: Der Ausdruck « nieder » bedeutet, dass entsprechend definierte Gruppen oder Verbindungen 1 bis 7, vorzugsweise 1 bis 4 Kohlenstoffatome enthalten.

Niederalkyl ist z. B. Methyl, Ethyl, n-Propyl, Isopropyl, n-Butyl, 2-Butyl, Isobutyl oder tert.-Butyl, ferner n-Pentyl, n-Hexyl oder n-Heptyl.

Niederalkoxy steht in erster Linie für Methoxy, Ethoxy, n-Propoxy, Isopropoxy, n-Butoxy, Isobutoxy oder tert.-Butoxy.

Niederalkylthio ist z. B. Methylthio oder Ethylthio.

Aryl steht, auch in Definitionen wie z. B. Arylniederalkyl, für aromatische Kohlenwasserstoffreste und

bedeutet z. B. 1- oder 2-Naphthyl, vorzugsweise jedoch Phenyl. Phenylniederalkyl ist z. B. Benzyl oder 1-oder 2-Phenylethyl.

Niederalkoxyaryl bedeutet z. B. Niederalkoxyphenyl und ist in erster Linie 2-, 3- oder 4-Methoxyphenyl.

Niederalkylen ist insbesondere $C_{2-6}$-Alkylen und steht z. B. für 1,2-Ethylen, 1,2- oder 1,3-Propylen, 1,3- oder 1,4-Butylen, 1,5-Pentylen oder 1,6-Hexylen, so dass die Gruppierung $R^2$—CH—N—$R^1$ z. B. den Pyrrolidinring bildet.

Mit Niederalkoxy substituiertes Niederalkylen ist insbesondere an den Kohlenstoffatomen substituiert, die nicht an Stickstoff gebunden sind, und bedeutet vorzugsweise 2-Methoxy-1,3-propylen.

Niederalkylen, das mit einem fünf- bis siebengliedrigen Carbocyclus kondensiert ist, ist insbesondere $C_{2-6}$-Alkylen, das mit einem gesättigten, partiell gesättigten oder aromatischen Carbocyclus kondensiert ist, wie z. B. mit Cyclopentyl, Cyclohexyl, Cycloheptyl, Cyclohexenyl oder Phenyl, so dass die Gruppierung $R^2$—CH—N—$R^1$ z. B. den Indolinring bildet.

Salze sind z. B. therapeutisch verwendbare Salze, z. B. Metall- oder Ammoniumsalze der Säure der Formel I, insbesondere Alkalimetall- oder Erdalkalimetallsalze, z. B. Natrium-, Kalium-, Magnesium- oder Calciumsalze, in erster Linie leicht kristallisierende Ammoniumsalze. Diese werden abgeleitet von Ammoniak oder organischen Aminen, z. B. Mono-, Di- oder Tri-nieder-(alkyl, cycloalkyl oder hydroxyalkyl)-aminen, Niederalkylendiaminen oder (Hydroxy-niederalkyl oder Aryl-niederalkyl)-niederalkylammonium Basen, z. B. Methylamin, Diethylamin, Triethylamin, Dicyclohexylamin, Triethanolamin, Ethylendiamin, Tris-(hydroxymethyl)-aminomethan oder Benzyl-trimethylammoniumhydroxid. Die Verbindung der Formel I bildet auch Säureadditionssalze. Diese werden z. B. mit solchen Säuren hergestellt, die therapeutisch verwendbare Säureadditionssalze ergeben. Das sind z. B. anorganische Säuren, wie Halogenwasserstoffsäuren, z. B. Chlorwasserstoff- oder Bromwasserstoffsäure, oder Schwefel-, Phosphor-, Salpeter- oder Perchlorsäure ; oder vorzugsweise organische Säuren, wie aliphatische oder aromatische Carbon- oder Sulfonsäuren, z. B. Ameisen-, Essig-, Propion-, Bernstein-, Glykol-, Milch-, Aepfel-, Wein-, Citronen-, Malein-, Fumar-, Hydroxymalein-, Brenztrauben-, Phenylessig-, Benzoe-, 4-Aminobenzoe-, Anthranil-, 4-Hydroxybenzoe-, Salicyl-, 4-Aminosalicyl-, Pamoe-, Nicotin-, Methansulfon-, Ethansulfon-, Hydroxyethansulfon-, Ethylensulfon-, Halogenbenzolsulfon-, Toluolsulfon-, Naphthalinsulfon-, Sulfanil- oder Cyclohexylsulfaminsäure ; oder die Ascorbinsäure. Ferner fallen auch pharmazeutisch ungeeignete Salze unter den Umfang dieser Erfindung, wie z. B. Picrate.

Die Erfindung betrifft besonders ein Verfahren zur Herstellung von Verbindungen der Formel I und ihrer Salze, dadurch gekennzeichnet, dass man von einer Verbindung der Formel II ausgeht, worin $R^1$ Wasserstoff, Niederalkyl oder Phenylniederalkyl bedeutet, $R^2$ für unsubstituiertes oder mit Niederalkoxy, Niederalkylthio, Aryl oder Niederalkoxyaryl substituiertes Niederalkyl, oder für Aryl steht, oder worin $R^1$ und $R^2$ gemeinsam $C_{3-4}$-Alkylen bedeuten, das mit Niederalkoxy substituiert oder mit einem fünf- bis siebengliedrigen Carbocyclus kondensiert sein kann, wobei Nieder einen Gehalt von 1-7 C-Atomen angibt und Aryl für 1- oder 2-Naphthyl oder Phenyl steht, und worin das Symbol * ein einheitlich in S- oder R-Konfiguration vorliegendes Kohlenstoffatom kennzeichnet.

Die Erfindung betrifft ganz besonders ein Verfahren zur Herstellung von Verbindungen der Formel I und ihrer Salze, dadurch gekennzeichnet, dass man von einer Verbindung der Formel II ausgeht, worin $R^1$ Wasserstoff, Methyl oder Benzyl bedeutet und $R^2$ für $C_1$-$C_7$-Alkyl oder Phenyl steht, oder worin $R^1$ und $R^2$ gemeinsam für 1,3-Propylen stehen, das mit einem fünf- oder sechsgliedrigen carbocyclus kondensiert sein kann, und worin das Symbol * ein einheitlich in S- oder R-Konfiguration vorliegendes Kohlenstoffatom kennzeichnet.

Die Erfindung betrifft in erster Linie ein Verfahren zur Herstellung von Verbindungen der Formel I und ihrer Salze, dadurch gekennzeichnet, dass man von einer Verbindung der Formel II ausgeht, worin die Gruppierung $R^2$—CH—N—$R^1$ den S- oder R-Pyrrolidin- oder S- oder R-Indolinring bedeutet. Dabei ist die Verwendung derjenigen Ausgangsverbindungen der Formel II bevorzugt, in denen das den Rest $R^2$ tragende Kohlenstoffatom die S-Konfiguration aufweist.

Das erfindungsgemässe Verfahren lässt sich gedanklich aufteilen in eine Cyclisierung (Bildung von III aus II) und eine Hydrolyse (Bildung von I aus III). Die Verfahrensschritte werden in an sich bekannter Weise wie folgt durchgeführt :

Cyclisierung

Ein 3-Methylen-2,5-diketopiperazin der Formel II wird in einem inerten Lösungsmittel, vorzugsweise unter Schutzgas, photochemisch cyclisiert. Geeignete inerte Lösungsmittel sind z. B. Kohlenwasserstoffe, wie Hexan, Toluol oder insbesondere Benzol, halogenierte Kohlenwasserstoffe, wie Chloroform, Dichlormethan, Niederalkancarbonitrile, wie Acetonitril, oder Niederalkanole, wie Methanol, Ethanol, 2-Propanol oder bevorzugtermassen tert.-Butanol. Als Schutzgase kommen z. B. Argon oder Stickstoff in Frage. Die photochemische Energiezufuhr erfolgt vorteilhafterweise mit einer Quecksilbermitteldruckdampflampe, beispielsweise für den Labormassstab mit einer HPK 125 Watt der Fa. Phillips, einer Osram XBO, 450 Watt, oder einer Ace-Hanovia, 450 Watt. Die Reaktionszeiten liegen bei Umsetzung von z. B. 0,01-0,1 Mol etwa zwischen 0,25 und 72 Stunden. Die Reaktionstemperaturen liegen je nach Lösungsmittel bei 0 bis 80 °C. Um zu starkes Erwärmen zu verhindern, wird der Ansatz gegebenenfalls gekühlt. Je

nach Grössenordnung der Umsatzmengen können auch stärkere Energiequellen verwendet werden.

Hydrolyse

Eine wie vorstehend durch Cyclisierung erhaltene Verbindung der Formel III wird durch Hydrolyse der beiden Amidgruppen des Diketopiperazins in die gewünschte Verbindung der Formel I und die leicht abtrennbare Aminosäure der Formel IV

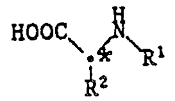 (IV)

gespalten. Die Hydrolyse erfolgt in der für Amidhydrolysen üblichen Weise (siehe z. B. Tetrahedron Letters 46, 4483 (1979)), vorzugsweise durch Einwirken starker Säuren, wie Halogenwasserstoffsäuren, z. B. Chlorwasserstoffsäure, bei erhöhter Temperatur, z. B. 80-150 °C. Die Trennung der beiden Aminosäuren I und IV lässt sich nach herkömmlichen dafür geeigneten Trennverfahren durchführen, wie z. B. nach chromatographischen Trennverfahren.

Je nach den Verfahrensbedingungen kann die Verbindung der Formel I als freie Verbindung, die, da es sich um eine Aminosäure handelt, als inneres Salz vorliegt, oder als Metall- bzw. Ammoniumsalz oder als Säureadditionssalz erhalten werden. Die Umwandlungen der freien Verbindung der Formel I in ein Salz oder eines Salzes in ein anderes Salz oder in die freie Verbindung der Formel I können in an sich bekannter Weise durchgeführt werden.

Bei einer besonders bevorzugten Verfahrensvariante steht die Gruppierung $R^2$—CH—N—$R^1$ in den Formeln II, III und IV für den 2S- oder 2R-Pyrrolidinring oder den 2S- oder 2R-Indolinring. In den beiden letztgenannten Fällen führt die Hydrolyse der Verbindung der Formel III zu zwei Aequivalenten der Verbindung der Formel I, da dann Formel I und IV identisch sind. Dieser Umstand bringt es mit sich, dass eine Abtrennung des Hydrolyseproduktes IV von I, wie in allen anderen Fällen notwendig, hier entfällt.

Das Verfahren umfasst auch diejenigen Ausführungsformen, wonach Zwischenprodukte isoliert und die restlichen Verfahrensschritte mit diesen durchgeführt werden oder die Ausgangsstoffe in situ hergestellt und ohne Isolierung verwendet werden oder das Verfahren auf irgendeiner Stufe abgebrochen wird; ferner können Ausgangsstoffe in Form von Derivaten verwendet oder während der Reaktion gebildet werden.

Die Ausgangsverbindungen der Formel II sind neu und bilden einen weiteren Gegenstand der vorliegenden Erfindung. Es gelten die gleichen Definitionen für $R^1$, $R^2$ und * wie angegeben, insbesondere auch die als «besonders», «ganz besonders» und «in erster Linie» bedeutsam herausgestellten Definitionen.

Ebenso sind die Zwischenprodukte der Formel III neu, mit Ausnahme des Cyclodipeptides der Indolincarbonsäure, und bilden insoweit einen weiteren Gegenstand der Erfindung. Auch hierfür gelten die gleichen Definitionen für $R^1$, $R^2$ und * wie angegeben, insbesondere auch die als «besonders», «ganz besonders» und «in erster Linie» bedeutsam herausgestellten Definitionen.

Die Verbindungen der Formel II lassen sich auf an sich bekannte Weise erhalten, so z. B. indem man ein Aminosäureanilid der Formel V in Gegenwart von Thionylchlorid in Dimethylformamid mit Brenztraubensäure der Formel VI kondensiert und dehydratisiert. Dabei werden die Verbindungen der Formel VII und VIII durchlaufen, die aber nicht isoliert werden müssen. Aminosäureanilide der Formel V sind aus Aminosäuren der Formel IV, die auf diese Weise rezykliert werden können, und Anilin auf konventionelle Weise erhältlich.

In den Verbindungen der Formeln V, VII und VIII haben die Symbole $R^1$, $R^2$ und * die unter Formel II angegebene Bedeutung.

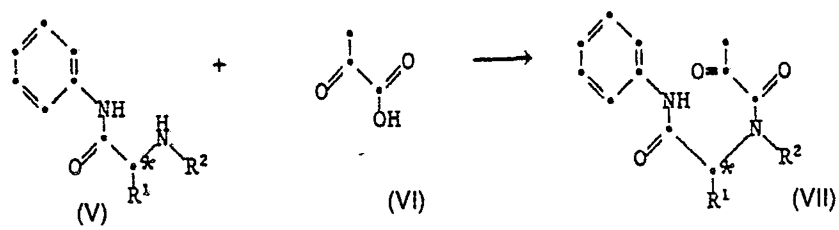

(V) (VI) (VII)

(VII) 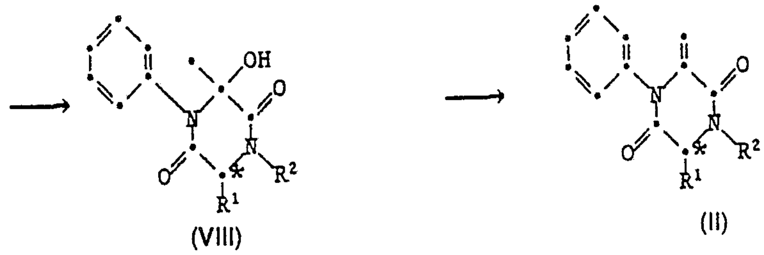

(VIII) (II)

Ein alternativer Zugang zu Verbindungen der Formel II besteht in der Kondensation von Brenztraubensäure der Formel VI mit einer Aminosäure der Formel IV und anschliessende Cyclisierung mit Anilin zu einer Verbindung der Formel VIII. Derartige Umsetzungen, die zu den Ausgangsverbindungen der Formel II führen, gehören zum Stand der Technik und bedürfen daher keiner weiteren Erläuterung.

Die folgenden Beispiele dienen zur Illustration der Erfindung. Temperaturen sind in Celsiusgraden angegeben.

Beispiel 1

2,42 g 3-Methylen-2-phenyl-2,3,6,7,8,8a-hexahydropyrrolo [1,2-a]-[8aS]-pyrazin-1,4-dion werden in 1 000 ml tert.-Butanol bei Raumtemperatur mit einer Quecksilbermitteldrucklampe (HPK 125 W der Firma Philips) 24 Stunden bestrahlt. Nach Abdestillieren des Lösungsmittels und Umkristallisieren aus Dichlormethan/Diethylether (1 : 1) erhält man das Cyclodipeptid aus S-Prolin und 2S-Indolincarbonsäure als helle Kristalle, Fp. 153°, $\alpha_{365nm}$ = — 107° (Ethanol).

1,2 g von diesem Cyclodipeptid werden bei 110° 12 Stunden in 6 M HCl erhitzt. Nach dem Abkühlen wird die Lösung mit Triethylamin neutralisiert und mit Chloroform extrahiert. Die organische Phase wird eingeengt und der Rückstand über eine Kieselgelsäule mit t.-Butanol/Wasser/Essigsäure (4 : 1 : 1) chromatographiert. Durch Eindampfen der Fraktionen, die ausschliesslich Material mit dem Rf-Wert 0,47 enthalten, erhält man reine 2S-Indolincarbonsäure, Fp. 175°, $\alpha_D^{20}$ = — 114° (c = 1 in 2 N HCl).

Das verwendete Ausgangsmaterial der Formel II lässt sich wie folgt herstellen :

17,6 g Brenztraubensäure in 30 ml Dimethylformamid p. A. werden auf 10° gekühlt, und bei dieser Temperatur werden innerhalb 20 Minuten 23,8 g Thionylchlorid zugegeben. Die orange Lösung wird eine Stunde bei 10° ausgerührt und anschliessend während 5 Minuten mit einer Lösung aus 19,0 g S-Prolinanilid und 80 ml Dichlormethan versetzt. Die Temperatur steigt trotz Eiskühlung auf ca. 20°. Die Lösung wird tiefrot. Sie wird auf 0-5° abgekühlt, anschliessend wird eine Lösung aus 20,2 g Triethylamin p. A. und 15 ml Dichlormethan zugetropft. Danach wird bei Raumtemperatur 2 Stunden gerührt. Die braunrote Lösung wird am Rotavap bei 70° Badtemperatur eingedampft und in 100 ml Wasser und 200 ml Dichlormethan aufgenommen. Die organische Phase wird zweimal mit je 50 ml Wasser und 100 ml Kochsalzlösung gewaschen. Die organische Phase wird eingedampft und getrocknet. Das rohe Kristallisat wird in 200 ml heissem Methanol gelöst und mit 3 g Aktivkohle versetzt, über Hyflo filtriert und abgekühlt. Nochmaliges Umkristallisieren der erhaltenen Kristalle aus Dichlormethan/Diethylether (1 : 1) liefert 3-Methylen-2-phenyl-2,3,6,7,8,8a-hexahydropyrrolo [1,2-a]-[8aS]-pyrazin-1,4-dion, Fp. 186°, $\alpha_{365nm}$ = 82° (Ethanol).

Beispiel 2

0,87 g 3-Methylen-2-phenyl-2,3,10,10a-tetrahydropyrazino [1,2-a]-[10aS]-indol-1,4-dion werden mit 350 ml tert.-Butanol und 35 ml Dimethylformamid versetzt. Die Suspension wird 30 Minuten mit Argon gespült und anschliessend während 24 Stunden mit einer Quecksilbermitteldrucklampe (HPK 125 W der Firma Phillips) in einer Quarzapparatur bestrahlt. Nach Abdestillieren des Lösungsmittels erhält man ein hellbraunes Kristallisat. Reinigung über eine Kieselgelsäule mit Essigester/Toluol (1 : 1) ergibt das Cyclodipeptid der 2S-Indolincarbonsäure als helles Kristallisat, Fp. 261-263°.

0,5 g dieses Cyclodipeptides werden in 10 ml 6N Salzsäure 15 Stunden auf 110° erwärmt. Nach dem Abkühlen wird mit 2N Natronlauge neutralisiert und die wässrige Phase mit Dichlormethan extrahiert. Die organische Phase wird abgetrennt und eingedampft. Man erhält 2S-Indolincarbonsäure als helles Kristallisat, Fp. 173-175°, $[\alpha]_D^{20}$ = — 113° in 2N HCl.

Das verwendete Ausgangsmaterial der Formel II lässt sich wie folgt erhalten :

16,3 g 2S-Indolincarbonsäure werden in 50 ml Wasser vorgelegt und mit ca. 25 ml 4N Natronlauge versetzt, so dass die Lösung pH = 9,5 aufweist. Dann wird auf 0° abgekühlt und innerhalb 30 Minuten Chlorkohlensäurebenzylester zugetropft. Der pH wird dabei durch entsprechende Zugabe von Natronlauge konstant gehalten. Nach dem Eintropfen wird noch 5 Stunden bei 0° gerührt, die Suspension auf Raumtemperatur erwärmt und die wässrige Phase mit Ether extrahiert. Die wässrige Phase wird anschliessend in eine Mischung von 200 g Eis, 40 ml konz. Salzsäure und 60 ml Wasser eingetragen. Das Produkt fällt aus, wird in Chloroform aufgenommen und getrocknet. Nach Umkristallisieren aus Ether/Petrolbenzin (Kp. 40°-60°) erhält man Benzyloxycarbonyl-2S-indolincarbonsäure, Fp. 116°.

18,6 g Benzyloxycarbonyl-2S-indolincarbonsäure werden in 200 ml Essigester gelöst und auf — 20° gekühlt. Zu dieser Lösung gibt man 6,32 g N-Methylmorpholin und tropft anschliessend 6,87 g Chlorkohlensäureethylester so zu, dass die Temperatur nicht über — 15° ansteigt. Zur entstandenen Suspension werden bei — 20° 5,86 g Anilin getropft, die Mischung wird auf Raumtemperatur erwärmt und eine Stunde gerührt. Die Suspension wird filtriert, der Filterrückstand mit Wasser gewaschen und getrocknet. Man erhält N-Benzyloxycarbonyl-2S-indolincarbonsäureanilid, Fp. 222°.

5 g N-Benzyloxycarbonyl-2S-indolincarbonsäureanilid werden in 100 ml Dimethylformamid gelöst und mit 1 g Palladiumkatalysator (5 % Pd auf Aktivkohle) versetzt. Die Mischung wird unter Wasserstoff bei Raumtemperatur und Normaldruck im Schüttelkolben hydriert. Nach 6 Minuten beträgt die Wasserstoffaufnahme 98 % und die Hydrierung ist zum Stillstand gekommen. Der Katalysator wird abfiltriert, die

Lösung eingedampft und über Kieselgel (Laufmittel Toluol/Essigester) gereinigt. Nach dem Eindampfen erhält man 2S-Indolincarbonsäureanilid, Fp. 110°.

4,4 g Brenztraubensäure werden unter Eiskühlung mit 10 ml Dimethylformamid versetzt. Anschliessend werden bei 0-5° 6,3 g Thionylchlorid zugetropft und die Mischung 30 Minuten im Eisbad gerührt. In diese Lösung werden 5,95 g 2S-Indolincarbonsäureanilid, gelöst in 20 ml Dichlormethan, eingetropft und die Mischung 2 Stunden gerührt. Danach wird eine Lösung von 5,5 g Triethylamin in 5 ml Dichlormethan so zugegeben, dass die Innentemperatur + 5° nicht übersteigt. Die braune Suspension wird 30 Minuten gerührt und anschliessend abfiltriert. Der Filterrückstand wird mit 20 ml Ether gewaschen und das Filtrat bei einer Badtemperatur von 80° eingedampft. Der kristalline Rückstand wird mit 50 ml Essigester und 50 ml Ether gewaschen und im Vakuum getrocknet. Man erhält 3-Methylen-2-phenyl-2,3,10,10a-tetrahydropyrazino [1,2-a]-[10aS]-indol-[1,4-dion, Fp. 241-243°, als weisse Kristalle, $[\alpha]_{436} = 18{,}3°$ (c = 1, Ethanol).

Beispiel 3

0,8 g 3,4-Dimethyl-6-methylen-1-phenyl-(3S)-piperazin-2,5-dion werden in 350 ml t-Butanol gelöst und unter Stickstoff 24 Stunden mit einer Quecksilbermitteldrucklampe vom Typ HPK 125 der Firma Phillips bestrahlt. Nach Abdampfen des Lösungsmittels im Vakuum erhält man 2,3-Dimethyl-2,3,10,10a-tetrahydropyrazino [1,2-a]-[3S,10aS]-indol-1,4-dion, 250 MHz NMR ($CDCl_3$, Angaben : δ in ppm, Multiplizität, Anzahl von Protonen) : 1,6 (d, 3H) ; 3,08 (s, 3H) ; 3,4-3,6 (m, 2H) ; 4,0 (q, 1H) ; 4,8 (t, 1H) ; 7,1-7,3 (m, 3H) ; 8,05 (dd, 1H), $[\alpha]_D = + 20{,}1°$ (c = 1, Ethanol).

Die Hydrolyse des 2,3-Dimethyl-2,3,10,10a-tetrahydropyrazino [1,2-a]-[3S,10aS]-indol-1,4-dion zum S-Enantiomer der Formel I erfolgt analog zu Beispiel 1.

Das benötigte Ausgangsmaterial der Formel II lässt sich wie folgt erhalten : Analog zur Herstellung des Ausgangsmaterials im Beispiel 2 wird das N-Benzyloxycarbonyl-N-methyl-S-alaninanilid, Fp. 65°, hergestellt. Daraus lässt sich wiederum analog das N-Methyl-S-alaninanilid, Fp. 42°, erhalten.

5,28 g Brenztraubensäure werden in 10 ml Thionylchlorid gelöst und bei —15° mit 4,4 ml Thionylchlorid versetzt. Die gelbe Lösung wird 30 Minuten unter Eiskühlung gerührt und anschliessend mit 5,5 g N-Methyl-S-alaninanilid, gelöst in 20 ml Dichlormethan, versetzt. Die braune Suspension wird 2 Stunden gerührt und bei 0° mit 8,3 ml Triethylamin versetzt. Es entsteht eine braune Suspension. Die Mischung wird bei 80° Badtemperatur im Vakuum eingedampft, der Rückstand in 150 ml Chloroform aufgenommen und dreimal mit je 50 ml Wasser gewaschen. Die organische Phase wird getrocknet und über Kieselgel mit Toluol/Essigester (1 : 1) gereinigt. Man erhält 2,8 g 3,4-Dimethyl-6-methylen-1-phenyl-3S-piperazin-2,5-dion als zähflüssiges Oel. 250 MHz NMR ($CDCl_3$, Angaben : δ in ppm, Multiplizität, Anzahl von Protonen) : 1,66 (d, 3H) ; 3,1 (s, 3H) ; 4,15 (q, 1H) : 4,64 (s, 1H) ; 5,76 (s, 1H), 7,1-7,3 (m, 2H) ; 7,6-7,8 (m, 3H), $[\alpha]_D = 29{,}7°$ (c = 1, Ethanol).

Beispiel 4

2,42 g 3-Methylen-2-phenyl-2,3,6,7,8,8a-hexahydropyrrolo [1,2-a]-[8aR]-pyrazin-1,4-dion werden in 1 tert.-Butanol 24 Stunden mit einer Quecksilbermitteldrucklampe (HPK 125 W der Firma Phillips) bei Raumtemperatur unter Stickstoff bestrahlt. Nach Abziehen des Lösungsmittels und Umkristallisieren aus Dichlormethan/Diethylether (1 : 1) erhält man das Cyclodipeptid aus R-Prolin und 2R-Indolincarbonsäure als helles Kristallisat mit einem Schmelzpunkt von 151-153°, $[\alpha]_{365} = + 105°$ (Ethanol).

1,5 g von diesem Cyclodipeptid werden über Nacht in 20 ml 6N HCl bei 110° gekocht. Nach Abkühlen der Lösung wird mit Triethylamin neutralisiert und die wässrige Lösung filtriert. Das Filtrat wird mit 50 ml Dichlormethan extrahiert, und die organische Phase wird eingedampft. Die Trennung über 50 g Kieselgel mit Butanol/Eisessig/Wasser (4 : 1 : 1) liefert 2R-Indolincarbonsäure, $R_f = 0{,}47$ (R-Prolin : $R_f = 0{,}08$) ; Fp. 174-175° ; $[\alpha]_D^{20} = + 112°$ in 2N HCl.

Das verwendete Ausgangsmaterial der Formel II lässt sich wie folgt herstellen :

4,4 g Brenztraubensäure werden in 10 ml Dimethylformamid gelöst, die Lösung wird auf 10° gekühlt und mit 5,5 g Thionylchlorid versetzt. Die hellrote Lösung wird eine Stunde bei 10° gerührt und anschliessend mit einer Lösung aus 4,7 g R-Prolinanilid in 20 ml Dichlormethan versetzt. Die Temperatur steigt trotz Kühlung auf ca. 15° an. Nach Abkühlen auf 0-5° wird eine Lösung aus 5,05 g Triethylamin in 5 ml Dichlormethan zugetropft und anschliessend bei Raumtemperatur 2 Stunden gerührt. Die braunrote Mischung wird wird bei 70° Badtemperatur am Rotationsverdampfer eingedampft und in 25 ml Wasser und 50 ml Dichlormethan aufgenommen. Die organische Phase wird noch zweimal mit 20 ml Wasser und 20 ml konz. Kochsalzlösung gewaschen, anschliessend eingedampft und getrocknet. Das erhaltene rohe Kristallisat wird in 50 ml siedendem Methanol gelöst und mit 0,5 g Aktivkohle versetzt. Nach Filtration erhält man 3,2 g 3-Methylen-2-phenyl-2,3,6,7,8,8a-hexahydropyrrolo [1,2-a]-[8aR]-pyrazin-1,4-dion als helle Kristalle, Fp. 184-185° ; $[\alpha]_{365} = + 81°$ (Ethanol).

Beispiele 5-8

Unter den in Beispiel 1 für die Photocyclisierung angegebenen Bedingungen werden folgende Verbindungen der allgemeinen Formel III hergestellt.

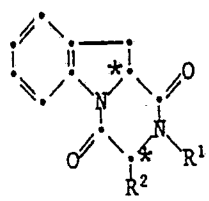

(III)

| Beispiel | $R^1$ | $R^2$ | Fp | $[\alpha]_D$; (c=1,Ethanol) |
|---|---|---|---|---|
| 5 | Methl | Isopropyl | 129° | +62° |
| 6 | Benzyl | Phenyl | 135° | +82° |
| 7 | Benzyl | Benzyl | Oel | |
| 8 | Methyl | Benzyl | Oel | |

Die Verbindungen der Beispiele 5 bis 8 werden in 6N Salzsäure 15 Stunden auf 110° erhitzt. Nach dem Einengen der Lösung werden die Hydrochloride in Methanol aufgenommen und mit 3 Aequivalenten Propylenoxid versetzt. Nach Abklingen der exothermen Reaktion wird die Lösung eingeengt. Die Trennung der erhaltenen Aminosäuren erfolgt wie in Beispiel 4 beschrieben.

Die Ausgangsmaterialien der allgemeinen Formel II werden nach der in Beispiel 1 angegebenen Methode hergestellt.

| $R^1$ | $R^2$ | Fp | $[\alpha]_D$; (c=1,Ethanol) |
|---|---|---|---|
| Methl | Isopropyl | Oel | |
| Benzyl | Phenyl | 152° | +136° |
| Benzyl | Benzyl | Oel | -211° |
| Methyl | Benzyl | Oel | |

Eine alternative Methode besteht in der Herstellung der Ausgangsmaterialien der allgemeinen Formel II aus den Zwischenprodukten der allgemeinen Formel VIII durch Erhitzen der Verbindungen der Formel VIII in Chloroform mit 10 % p-Toluolsulfonsäure als Katalysator.

Zu den Zwischenprodukten der allgemeinen Formel VIII gelangt man, wenn bei der Herstellung der Ausgangsmaterialien analog Beispiel I statt Triethylamin als Base Diisopropylaethylamin verwendet wird

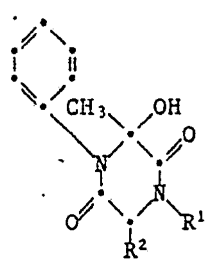

(VIII)

| $R^1$ | $R^2$ | Fp |
|---|---|---|
| Benzyl | Phenyl | 169° |
| Methyl | Methyl | 168-9° |
| 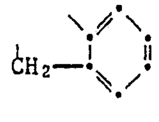 | | 212-13° |

Zu den Ausgangsmaterialien der allgemeinen Formel II gelangt man ausserdem ausgehend von den Zwischenprodukten der allgemeinen Formel VII durch Erhitzen in Chloroform mit 5 % p-Toluolsulfonsäure als Katalysator.

Die Herstellung der Zwischenprodukte der allgemeinen Formel VII ist nachfolgend beschrieben.

In eine Mischung aus 28,8 ml DMF und 40 ml Acetonitril werden bei — 30° 11,6 ml Oxalylchlorid eingetropft. Die Suspension wird bei — 25° noch 30 Min. gerührt und anschliessend mit 11 g Brenztraubensäure versetzt. Nach 30 Min. wird die Lösung aus 11,8 g Prolinanilid in 50 ml Methylenchlorid so zudosiert, dass die Innentemperatur nicht über — 15° steigt. Nach Abkühlen auf — 20° wird dann eine Lösung von 54 ml Triäthylamin in 50 ml Methylenchlorid zugegeben. Die Reaktionsmischung wird auf Raumtemperatur erwärmt und filtriert. Das Filtrat wird eingedampft und über Kieselgel mit Essigsäureaethylester/Methylenchlorid 10 : 1 gereinigt.

Man erhält farblose Kristalle der Formel

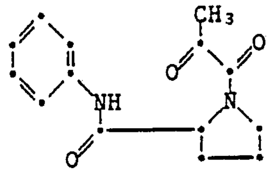

vom Schmelzpunkt 120° ; $[\alpha]_D$ = — 157° (c = 1, $CH_2Cl_2$).

**Patentansprüche**

1. Verfahren zur Herstellung von enantiomerenreinen Verbindungen der Formel I

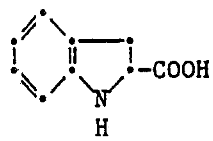

(I)

und ihrer Salze, dadurch gekennzeichnet, dass man eine Verbindung der Formel II,

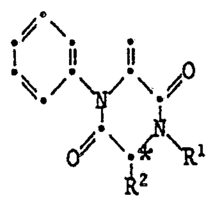

(II)

worin $R^1$ Wasserstoff, Niederalkyl oder Arylniederalkyl bedeutet, $R^2$ für unsubstituiertes oder mit Niederalkoxy, Niederalkylthio, Aryl oder Niederalkoxyaryl substituiertes Niederalkyl, oder für Aryl steht, oder worin $R^1$ und $R^2$ gemeinsam Niederalkylen bedeuten, das mit Niederalkoxy substituiert oder mit einem fünf- bis siebengliedrigen Carbocyclus kondensiert sein kann, wobei Nieder einen Gehalt von 1 bis 7 C-Atomen angibt und Aryl für 1- oder 2-Naphthyl oder Phenyl steht, und worin das Symbol * ein einheitlich in S- oder R-Konfiguration vorliegendes Kohlenstoffatom kennzeichnet, zu einer Verbindung der Formel III,

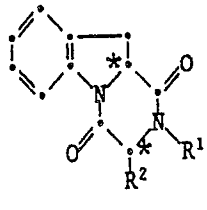

(III)

worin $R^1$ und $R^2$ die angegebenen Bedeutungen haben und die beiden Symbole * entweder beide ein in S-Konfiguration oder beide ein in R-Konfiguration vorliegendes Kohlenstoffatom kennzeichnen, diastereoselektiv photochemisch cyclisiert, diese Verbindung hydrolysiert und aus dem Hydrolysat die Verbindung der Formel I, gegebenenfalls in Form eines Salzes, abtrennt und gegebenenfalls eine erhaltene freie Verbindung der Formel I in ein Salz oder ein Salz in ein anderes Salz oder in die freie Verbindung der Formel I umwandelt.

2. Verfahren nach Anspruch 1 zur Herstellung von enantiomerenreinen Verbindungen der Formel I und ihrer Salze, dadurch gekennzeichnet, dass man von einer Verbindung der Formel II ausgeht, worin $R^1$ Wasserstoff, Niederalkyl oder Phenylniederalkyl bedeutet, $R^2$ für unsubstituiertes oder mit Niederalkoxy, Niederalkylthio, Aryl oder Niederalkoxyaryl substituiertes Niederalkyl, oder für Aryl steht, oder worin $R^1$ und $R^2$ gemeinsam $C_{3-4}$-Alkylen bedeuten, das mit Niederalkoxy substituiert oder mit einem fünf- bis siebengliedrigen Carbocyclus kondensiert sein kann, wobei Nieder einen Gehalt von 1 bis 7 C-Atomen angibt und Aryl für 1- oder 2-Naphthyl oder Phenyl steht, und worin das Symbol * ein einheitlich in S- oder R-Konfiguration vorliegendes Kohlenstoffatom kennzeichnet.

3. Verfahren nach Anspruch 1 zur Herstellung von enantiomerenreinen Verbindungen der Formel I und ihrer Salze, dadurch gekennzeichnet, dass man von einer Verbindung der Formel II ausgeht, worin $R^1$ Wasserstoff, Methyl oder Benzyl bedeutet und $R^2$ für $C_1$-$C_7$-Alkyl oder Phenyl steht, oder worin $R^1$ und $R^2$ gemeinsam für 1,3-Propylen stehen, das mit einem fünf- oder sechsgliedrigen Carbocyclus kondensiert sein kann, und worin das Symbol * ein einheitlich in S- oder R-Konfiguration vorliegendes Kohlenstoffatom kennzeichnet.

4. Verfahren nach Anspruch 1 zur Herstellung von enantiomerenreinen Verbindungen der Formel I und ihrer Salze, dadurch gekennzeichnet, dass man von einer Verbindung der Formel II ausgeht, worin die Gruppierung $R^2$—CH—N—$R^1$ den S- oder R-Pyrrolidin- oder S- oder R-Indolinring bedeutet.

5. Verfahren nach Anspruch 1 zur Herstellung von enantiomerenreinen Verbindungen der Formel I und ihrer Salze, dadurch gekennzeichnet, dass man von einer Verbindung der Formel II ausgeht, worin die Gruppierung $R^2$—CH—N—$R^1$ den S-Pyrrolidin- oder S-Indolinring bedeutet.

6. Verbindungen der Formel II,

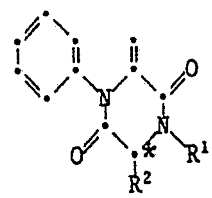

(II)

worin $R^1$ Wasserstoff, Niederalkyl oder Arylniederalkyl bedeutet, $R^2$ für unsubstituiertes oder mit Niederalkoxy, Niederalkylthio, Aryl oder Niederalkoxyaryl substituiertes Niederalkyl, oder für Aryl steht, oder worin $R^1$ und $R^2$ gemeinsam Niederalkylen bedeuten, das mit Niederalkoxy substituiert oder mit einem fünf- bis siebengliedrigen Carbocyclus kondensiert sein kann, wobei Nieder einen Gehalt von 1-7 C-Atomen angibt und Aryl für 1- oder 2-Naphthyl oder Phenyl steht, und worin das Symbol * ein einheitlich in S- oder R-Konfiguration vorliegendes Kohlenstoffatom kennzeichnet, und Enantiomerengemische und Salze davon.

7. Verbindungen der Formel II nach Anspruch 6, worin $R^1$ Wasserstoff, Niederalkyl oder Phenylniederalkyl bedeutet, $R^2$ für unsubstituiertes oder mit Niederalkoxy, Niederalkylthio, Aryl oder Niederalkoxyaryl substituiertes Niederalkyl, oder für Aryl steht, oder worin $R^1$ und $R^2$ gemeinsam $C_{3-4}$-Alkylen bedeuten, das mit Niederalkoxy substituiert oder mit einem fünf- bis siebengliedrigen Carbocyclus kondensiert sein kann, wobei Nieder einen Gehalt von 1 bis 7 C-Atomen angibt und Aryl für 1- oder 2-Naphthyl oder Phenyl steht, und worin das Symbol * ein einheitlich in S- oder R-Konfiguration vorliegendes Kohlenstoffatom kennzeichnet, und Enantiomerengemische und Salze davon.

8. Verbindungen der Formel II nach Anspruch 6, worin $R^1$ Wasserstoff, Methyl oder Benzyl bedeutet und $R^2$ für $C_1$-$C_7$-Alkyl oder Phenyl steht, oder worin $R^1$ und $R^2$ gemeinsam für 1,3-Propylen stehen, das mit einem fünf- oder sechsgliedrigen Carbocyclus kondensiert sein kann, und worin das Symbol * ein einheitlich in S- oder R-Konfiguration vorliegendes Kohlenstoffatom kennzeichnet, und Enantiomerengemische und Salze davon.

9. Verbindungen der Formel II nach Anspruch 6, worin die Gruppierung $R^2$—CH—N—$R^1$ den S- oder R-Pyrrolidin- oder S- oder R-Indolinring bedeutet, und Enantiomerengemische und Salze davon.

10. Verbindungen der Formel III,

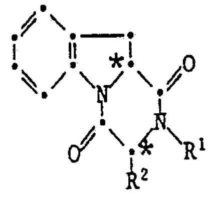

(III)

worin $R^1$ Wasserstoff, Niederalkyl oder Arylniederalkyl bedeutet, $R^2$ für unsubstituiertes oder mit Niederalkoxy, Niederalkylthio, Aryl oder Niederalkoxyaryl substituiertes Niederalkyl, oder für Aryl steht, oder worin $R^1$ und $R^2$ gemeinsam Niederalkylen bedeuten, das mit Niederalkoxy substituiert oder mit

einem fünf- bis siebengliedrigen Carbocyclus kondensiert sein kann, wobei Nieder einen Gehalt von 1 bis 7 C-Atomen angibt und Aryl für 1- oder 2-Naphthyl oder Phenyl steht, und worin das Symbol * ein einheitlich in S- oder R-Konfiguration vorliegendes Kohlenstoffatom kennzeichnet, und Enantiomerengemische und Salze davon, mit Ausnahme des Cyclodipeptids der Indolincarbonsäure.

11. Verbindungen der Formel III nach Anspruch 10, worin $R^1$ Wasserstoff, Niederalkyl oder Phenylniederalkyl bedeutet, $R^2$ für unsubstituiertes oder mit Niederalkoxy, Niederalkylthio, Aryl oder Niederalkoxyaryl substituiertes Niederalkyl, oder für Aryl steht, oder worin $R^1$ und $R^2$ gemeinsam $C_{3-4}$-Alkylen bedeuten, das mit Niederalkoxy substituiert oder mit einem fünf- bis siebengliedrigen Carbocyclus kondensiert sein kann, wobei Nieder einen Gehalt von 1 bis 7 C-Atomen angibt und Aryl für 1- oder 2-Naphthyl oder Phenyl steht, und worin das Symbol * ein einheitlich in S- oder R-Konfiguration vorliegendes Kohlenstoffatom kennzeichnet, und Enantiomerengemische und Salze davon, mit Ausnahme des Cyclodipeptids der Indolincarbonsäure.

12. Verbindungen der Formel III nach Anspruch 10, worin $R^1$ Wasserstoff, Methyl oder Benzyl bedeutet und $R^2$ für $C_1$-$C_2$-Alkyl oder Phenyl steht, oder worin $R^1$ und $R^2$ gemeinsam für 1,3-Propylen stehen, das mit einem fünf- oder sechsgliedrigen Carbocyclus kondensiert sein kann, und worin das Symbol * ein einheitlich in S- oder R-Konfiguration vorliegendes Kohlenstoffatom kennzeichnet, und Enantiomerengemische und Salze davon, mit Ausnahme des Cyclodipeptids der Indolincarbonsäure.

13. Verbindungen der Formel III nach Anspruch 10, worin die Gruppierung $R^2$—CH—N—$R^1$ den S- oder R-Pyrrolidinring bedeutet, und Enantiomerengemische und Salze davon.

**Claims**

1. A process for the preparation of an optically pure compound of the formula I

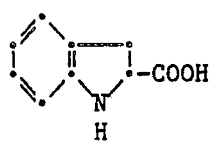

(I)

or a salt thereof, which comprises diastereoselectively cyclising a compound of the formula II

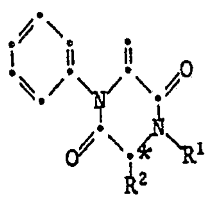

(II)

wherein $R^1$ is hydrogen, lower alkyl or aryl-lower alkyl, $R^2$ is lower alkyl which is unsubstituted or substituted by lower alkoxy, lower alkylthio, aryl or lower alkoxyaryl, or is aryl, or wherein $R^1$ and $R^2$, when taken together, are lower alkylene which may be substituted by lower alkoxy or fused to a 5- to 7-membered carbocyclic ring, lower signifying a content of 1 to 7 carbon atoms and aryl being 1- or 2-naphthyl or phenyl, and wherein the symbol * denotes a carbon atom which is always either in the S or in the R configuration, by photochemical means, to give a compound of the formula III

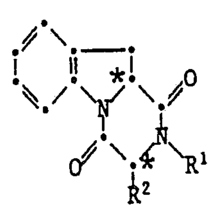

(III)

wherein $R^1$ and $R^2$ have the given meanings and each symbol * denotes a carbon atom, with both carbon atoms together being either in the S configuration or in the R configuration, hydrolysing said compound and isolating the compound of the formula I, optionally in the form of a salt, from the hydrolysate, and, if desired, converting a resultant free compound of the formula I into a salt or converting a salt into another salt or into the free compound of the formula I.

2. A process according to claim 1 for the preparation of an optically pure compound of the formula I or a salt thereof, which comprises starting from a compound of the formula II, wherein $R^1$ is hydrogen, lower alkyl or phenyl-lower alkyl, $R^2$ is lower alkyl which is unsubstituted or substituted by lower alkoxy,

lower alkylthio, aryl or lower alkoxyaryl, or is aryl, or wherein $R^1$ and $R^2$, when taken together, are $C_3$-$C_4$alkylene which may be substituted by lower alkoxy or fused to a 5- to 7-membered carbocyclic ring, lower signifying a content of 1 to 7, carbon atoms and aryl 1- or 2-naphthyl or phenyl, and wherein the symbol * denotes a carbon atom which is always either in the S or in the R configuration.

3. A process according to claim 1 for the preparation of an optically pure compound of the formula I or a salt thereof, which comprises starting from a compound of the formula II, wherein $R^1$ is hydrogen, methyl or benzyl, and $R^2$ is $C_1$-$C_7$alkyl or phenyl, or wherein $R^1$ and $R^2$, when taken together, are 1,3-propylene which may be fused to a 5- or 6-membered carbocyclic ring and wherein the symbol * denotes a carbon atom which is always either in the S or in the R configuration.

4. A process according to claim 1 for the preparation of an optically pure compound of the formula I or a salt thereof, which comprises starting from a compound of the formula II, wherein the $R^2$—CH—N—$R^1$ grouping denotes the S- or R-pyrrolidine ring or the S- or R-indoline ring.

5. A process according to claim 1 for the preparation of an optically pure compound of the formula I or a salt thereof, which comprises starting from a compound of the formula II, wherein the $R^2$—CH—N—$R^1$ grouping denotes the S-pyrrolidine or S-indoline ring.

6. A compound of the formula II

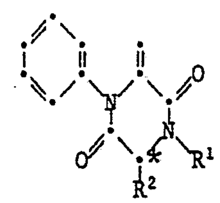

(II)

wherein $R^1$ is hydrogen, lower alkyl or aryl-lower alkyl, $R^2$ is lower alkyl which is unsubstituted or substituted by lower alkoxy, lower alkylthio, aryl or lower alkoxyaryl, or is aryl, or wherein $R^1$ and $R^2$, when taken together, are lower alkylene which may be substituted by lower alkoxy or fused to a 5- to 7-membered carbocyclic ring, lower signifying a content of 1 to 7 carbon atoms and aryl being 1- or 2-naphthyl or phenyl, and wherein the symbol * denotes a carbon atom which is always either in the S or in the R configuration, or a mixture of enantiomers or a salt thereof.

7. A compound of the formula II according to claim 6, wherein $R^1$ is hydrogen, lower alkyl or phenyl-lower alkyl, $R^2$ is lower alkyl which is unsubstituted or substituted by lower alkoxy, lower alkylthio, aryl or lower alkoxyaryl, or is aryl, or wherein $R^1$ and $R^2$, when taken together, are $C_3$-$C_4$alkylene which may be substituted by lower alkoxy or fused to a 5- to 7-membered carbocyclic ring, lower signifying a content of 1 to 7 carbon atoms and aryl being 1- or 2-naphthyl or phenyl, and wherein the symbol * denotes a carbon atom which is always either in the S or in the R configuration, or a mixture of enantiomers or a salt thereof.

8. A compound of the formula II according to claim 6, wherein $R^1$ is hydrogen methyl or benzyl, and $R^2$ is $C_1$-$C_7$alkyl or phenyl, or wherein $R^1$ and $R^2$, when taken together, are 1,3-propylene which may be fused to a 5- or 6-membered carbocyclic ring and wherein the symbol * denotes a carbon atom which is always either in the S or in the R configuration, or a mixture of enantiomers or a salt thereof.

9. A compound of the formula II according to claim 6, wherein the $R^2$—CH—N—$R^1$ grouping denotes the S- or R-pyrrolidine ring or the S- or R-indoline ring, or a mixture of enantiomers or a salt thereof.

10. A compound of the formula III

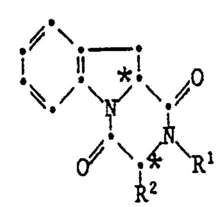

(III)

wherein $R^1$ is hydrogen, lower alkyl or aryl-lower alkyl, $R^2$ is lower alkyl which is unsubstituted or substituted by lower alkoxy, lower alkylthio, aryl or lower alkoxyaryl, or is aryl, or wherein $R^1$ and $R^2$, when taken together, are lower alkylene which may be substituted by lower alkoxy or fused to a 5- to 7-membered carbocyclic ring, lower signifying a content of 1 to 7 carbon atoms and aryl being 1- or 2-naphthyl or phenyl, and wherein the symbol * denotes a carbon atom which is always either in the S or in the R configuration, or a mixture of enantiomers or a salt thereof, except the cyclodipeptide of indolinecarboxylic acid.

11. A compound of the formula III according to claim 10, wherein $R^1$ is hydrogen, lower alkyl or phenyl-lower alkyl, $R^2$ is lower alkyl which is unsubstituted or substituted by lower alkoxy, lower alkylthio, aryl or lower alkoxyaryl, or is aryl, or wherein $R^1$ and $R^2$, when taken together, are $C_3$-$C_4$alkylene which

may be substituted by lower alkoxy or fused to a 5- to 7-membered carbocyclic ring, lower signifying a content of 1 to 7 carbon atoms and aryl being 1- or 2-naphthyl or phenyl, and wherein the symbol * denotes a carbon atom which is always either in the S or in the R configuration, or a mixture of enantiomers or a salt thereof, except the cyclodipeptide of indolinecarboxylic acid.

12. A compound of the formula III according to claim 10, wherein $R^1$ is hydrogen, methyl or benzyl, and $R^2$ is $C_1$-$C_7$alkyl or phenyl, or wherein $R^1$ and $R^2$, when taken together, are 1,3-propylene which may be fused to a 5- or 6-membered carbocyclic ring and wherein the symbol * denotes a carbon atom which is always either in the S or in the R configuration, or a mixture of enantiomers or a salt thereof, except the cyclodipeptide of indolinecarboxylic acid.

13. A compound of the formula III according to claim 10, wherein the $R^2$—CH—N—$R^1$ grouping denotes the S- or R-pyrrolidine ring, or a mixture of enantiomers or a salt thereof.

**Revendications**

1. Procédé pour préparer des énantiomères purs du composé de formule I :

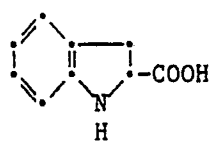

(I)

et les sels correspondants, procédé caractérisé en ce qu'on cyclise diastéréosélectivement, par voie photochimique, un composé répondant à la formule II :

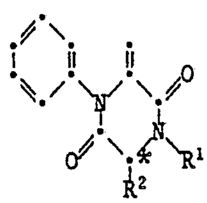

(II)

dans laquelle $R^1$ représente l'hydrogène, un alkyle inférieur ou un arylalkyle à alkyle inférieur, $R^2$ représente un alkyle inférieur non substitué ou porteur d'un alcoxy inférieur, d'un alkylthio inférieur, d'un aryle ou d'un alcoxy-aryle à alcoxy inférieur, ou représente un aryle, ou encore $R^1$ et $R^2$ forment ensemble un radical alkylène inférieur qui peut porter un alcoxy inférieur ou qui peut être condensé à un carbocycle comportant de 5 à 7 maillons, le qualificatif « inférieur » voulant dire ici que le nombre d'atomes de carbone est compris entre 1 et 7, et le vocable « aryle » désignant un radical naphtyle-1, naphtyle-2 ou phényle, et dans laquelle l'astérisque * indique que l'atome de carbone qui le porte a uniquement la configuration S ou la configuration R, cyclisation qui conduit à un composé répondant à la formule III :

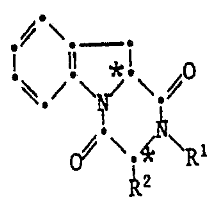

(III)

dans laquelle $R^1$ et $R^2$ ont les significations précédemment données et dans laquelle les deux astérisques * indiquent que les atomes de carbone correspondants ont soit tous les deux la configuration S soit tous les deux la configuration R, on hydrolyse ce composé et, à partir de l'hydrolysat, on sépare le composé de formule I, éventuellement sous la forme d'un sel, et, le cas échéant, si l'on a obtenu un composé libre de formule I, on le transforme en un sel, ou, si l'on a obtenu un sel, on transforme celui-ci en un autre sel ou en le composé libre de formule I.

2. Procédé selon la revendication 1 pour la préparation d'énantiomères purs du composé de formule I et de ses sels, procédé caractérisé en ce qu'on part d'un composé répondant à la formule II dans laquelle $R^1$ représente l'hydrogène, un alkyle inférieur ou un phénylalkyle à alkyle inférieur, $R^2$ représente un alkyle inférieur non substitué ou porteur d'un alcoxy inférieur, d'un alkylthio inférieur, d'un aryle ou d'un alcoxyaryle à alcoxy inférieur, ou représente un aryle, ou encore $R^1$ et $R^2$ forment ensemble un alkylène à 3 ou 4 atomes de carbone qui peut porter un alcoxy inférieur ou être condensé à un carbocycle comportant de 5 à 7 maillons, le qualificatif « inférieur » voulant dire que le nombre d'atomes de carbone

est compris entre 1 et 7, et le terme « aryle » désignant un radical napthyle-1, naphtyle-2 ou phényle, et dans laquelle le symbole * veut dire que l'atome de carbone qui le porte a uniquement l'une des deux configurations S et R.

3. Procédé selon la revendication 1 pour la préparation d'énantiomères purs du composé de formule I et de ses sels, procédé caractérisé en ce qu'on part d'un composé répondant à la formule II dans laquelle $R^1$ représente l'hydrogène, un méthyle ou un benzyle, $R^2$ représente un alkyle en $C_1$-$C_7$ ou un phényle, ou $R^1$ et $R^2$ forment ensemble un radical propylène-1,3 éventuellement condensé à un carbocycle comportant 5 ou 6 maillons, et dans laquelle le symbole * veut dire que l'atome de carbone qui le porte présente uniquement l'une des deux configurations S et R.

4. Procédé selon la revendication 1 pour la préparation d'énantiomères purs du composé de formule I et de ses sels, procédé caractérisé en ce qu'on part d'un composé répondant à la formule II dans laquelle le groupement $R^2$—CH—N—$R^1$ représente le cycle de la pyrrolidine S ou R ou le cycle de l'indoline S ou R.

5. Procédé selon la revendication 1 pour la préparation d'énantiomères purs du composé de formule I et de ses sels, procédé caractérisé en ce qu'on part d'un composé répondant à la formule II dans laquelle le groupement $R^2$—CN—N—$R^1$ représente le cycle de la pyrrolidine S ou de l'indoline S.

6. Composés répondant à la formule II :

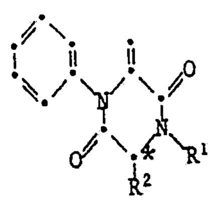

(II)

dans laquelle $R^1$ représente l'hydrogène, un alkyle inférieur ou un arylalkyle à alkyle inférieur, $R^2$ représente un alkyle inférieur non substitué ou porteur d'un alcoxy inférieur, d'un alkylthio inférieur, d'un aryle ou d'un alcoxy-aryle à alcoxy inférieur, ou représente un aryle, ou encore $R^1$ et $R^2$ forment ensemble un radical alkylène inférieur qui peut porter un alcoxy inférieur ou qui peut être condensé à un carbocycle comportant de 1 à 7 maillons, le qualificatif « inférieur » voulant dire ici que le nombre d'atomes de carbone est compris entre 1 et 7, et le vocable « aryle » désignant un radical naphtyle-1, naphtyle-2 ou phényle, et dans laquelle le symbole * veut dire que l'atome de carbone qui le porte a uniquement la configuration S ou uniquement la configuration R, ainsi que les mélanges d'énantiomères et les sels de ces composés.

7. Composés selon la revendication 6 qui répondent à la formule II dans laquelle $R^1$ représente l'hydrogène, un alkyle inférieur ou un phénylalkyle à alkyle inférieur, $R^2$ représente un alkyle inférieur non substitué ou porteur d'un alcoxy inférieur, d'un alkylthio inférieur, d'un aryle ou d'un alcoxy-aryle à alcoxy inférieur, ou représente un aryle, ou dans laquelle $R^1$ et $R^2$ forment ensemble un alkylène à 3 ou 4 atomes de carbone qui peut porter un alcoxy inférieur ou être condensé à un carbocycle comportant de 5 à 7 maillons, le qualificatif « inférieur » signifiant que le nombre d'atomes de carbone est compris entre 1 et 7 et le vocable « aryle » désignant un radical naphtyle-1, naphtyle-2 ou phényle, et dans laquelle le symbole * veut dire que l'atome de carbone qui le porte a uniquement la configuration S ou la configuration R, ainsi que les mélanges d'énantiomères et les sels de ces composés.

8. Composés selon la revendication 6 qui répondent à la formule II dans laquelle $R^1$ représente l'hydrogène, un méthyle ou un benzyle et $R^2$ un alkyle en $C_1$-$C_7$ ou un phényle, ou dans laquelle $R^1$ et $R^2$ forment ensemble un radical propylène-1,3 qui peut être condensé à un carbocycle comportant 5 ou 6 maillons, et dans laquelle le symbole * veut dire que l'atome de carbone qui le porte a uniquement l'une des deux configurations S et R, ainsi que les mélanges d'énantiomères et les sels de ces composés.

9. Composés selon la revendication 6 qui répondent à la formule II dans laquelle le groupement $R^2$—CH—N—$R^1$ représente le cycle de la pyrrolidine S ou R ou le cycle de l'indoline S ou R, ainsi que les mélanges d'énantiomères et les sels de ces composés.

10. Composés répondant à la formule III :

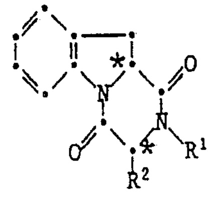

(III)

dans laquelle $R^1$ représente l'hydrogène, un alkyle inférieur ou un arylalkyle à alkyle inférieur, $R^2$ représente un alkyle inférieur non substitué ou porteur d'un alcoxy inférieur, d'un alkylthio inférieur, d'un

aryle ou d'un alcoxy-aryle à alcoxy inférieur, ou représente un aryle, ou encore $R^1$ et $R^2$ forment ensemble un radical alkylène inférieur qui peut porter un alcoxy inférieur ou qui peut être condensé à un carbocycle comportant de 1 à 7 maillons, le qualificatif « inférieur » voulant dire ici que le nombre d'atomes de carbone est compris entre 1 et 7, et le vocable « aryle » désignant un radical naphtyle-1, naphtyle-2 ou phényle, et dans laquelle le symbole * veut dire que l'atome de carbone qui le porte a uniquement la configuration S ou uniquement la configuration R, ainsi que les mélanges d'énantiomères et les sels de ces composés, à l'exception du cyclodipeptide de l'acide indoline-carboxylique.

11. Composés selon la revendication 10 qui répondent à la formule III dans laquelle $R^1$ représente l'hydrogène, un alkyle inférieur ou un phénylalkyle à alkyle inférieur, $R^2$ représente un alkyle inférieur non substitué ou porteur d'un alcoxy inférieur, d'un alkylthio inférieur, d'un aryle ou d'un alcoxy-aryle à alcoxy inférieur, ou représente un aryle, ou dans laquelle $R^1$ et $R^2$ forment ensemble un alkylène à 3 ou 4 atomes de carbone qui peut porter un alcoxy inférieur ou être condensé à un carbocycle comportant de 5 à 7 maillons, le qualificatif « inférieur » signifiant que le nombre d'atomes de carbone est compris entre 1 et 7 et le vocable « aryle » désignant un radical naphtyle-1, naphtyle-2 ou phényle, et dans laquelle le symbole * veut dire que l'atome de carbone qui le porte a uniquement la configuration S ou la configuration R, ainsi que les mélanges d'énantiomères et les sels de ces composés, à l'exception du cyclodipeptide de l'acide indoline-carboxylique.

12. Composés selon la revendication 10 qui répondent à la formule III dans laquelle $R^1$ représente l'hydrogène, un méthyle ou un benzyle et $R^2$ un alkyle en $C_1$-$C_7$ ou un phényle, ou dans laquelle $R^1$ et $R^2$ forment ensemble un radical propylène-1,3 éventuellement condensé à un carbocycle à 5 ou 6 maillons, et dans laquelle le symbole * signifie que l'atome de carbone qui le porte a uniquement la configuration S ou la configuration R, ainsi que les mélanges d'énantiomères et les sels de ces composés, à l'exception du cyclodipeptide de l'acide indoline-carboxylique.

13. Composés selon la revendication 10 qui répondent à la formule III dans laquelle le groupement $R^2$—CH—N—$R^1$ représente le cycle de la pyrrolidine S ou R, ainsi que les mélanges d'énantiomères et les sels de ces composés.